(19) Europäisches Patentamt
European Patent Office
Office européen des brevets

(11) **EP 1 205 174 B1**

(12) **EUROPÄISCHE PATENTSCHRIFT**

(45) Veröffentlichungstag und Bekanntmachung des Hinweises auf die Patenterteilung:
**11.08.2004 Patentblatt 2004/33**

(51) Int Cl.⁷: **A61K 7/06**, A61K 7/11, A61K 7/00

(21) Anmeldenummer: **01123409.3**

(22) Anmeldetag: **28.09.2001**

(54) **Volumengebendes und gezielt den Haaransatz festigendes Haarbehandlungsprodukt**

Volumising hair treatment composition providing targeted hold to the base of the hair

Produit capillaire apportant du volume à la chevelure et fixant spécifiquement le cheveu à sa base

(84) Benannte Vertragsstaaten:
**AT BE CH CY DE DK ES FI FR GB GR IE IT LI LU MC NL PT SE TR**

(30) Priorität: **10.11.2000 DE 10055935**

(43) Veröffentlichungstag der Anmeldung:
**15.05.2002 Patentblatt 2002/20**

(73) Patentinhaber: **Wella Aktiengesellschaft**
**64295 Darmstadt (DE)**

(72) Erfinder:
• **Kalbfleisch, Axel**
**64295 Darmstadt (DE)**
• **Birkel, Susanne**
**64285 Darmstadt (DE)**
• **Lede, Michael**
**63225 Langen (DE)**

(56) Entgegenhaltungen:
**EP-A- 0 172 713**      **EP-A- 1 075 832**
**EP-A- 1 110 536**      **WO-A-00/61084**
**DE-A- 19 907 715**      **DE-A- 19 937 434**

**Beschreibung**

Gegenstand der Erfindung ist ein Haarbehandlungsprodukt, mit dem es möglich ist, einer Frisur mehr Volumen zu verleihen und gezielt den Haaransatz zu festigen. Verwendet wird dabei eine wässrige, polymer- und tensidhaltige Zusammensetzung, welche mittels eines Aerosoltreibmittels bei der Anwendung aufgeschäumt wird, aber nicht über einen Schaumkopf abgegeben sondern über einen Haarspray-Sprühkopf versprüht wird und einen sehr schnell zusammenbrechenden, instabilen Sprühschaum bildet.

Haarbehandlungsmittel in Aerosolform werden im allgemeinen in zwei Gruppen eingeteilt: Aerosol-Haarsprays und Aerosol-Haarschäume. Aerosol-Haarsprays dienen dazu, unspezifisch auf die gesamte Frisur aufgesprüht zu werden und hierdurch die Frisur in der zuvor hergestellten Form zu fixieren. Ein gezieltes Stylen einzelner Haarpartien ist so nicht möglich. Aerosol-Schäume werden zunächst auf die Hand aufgebracht und anschließend wird der Schaum mit den Händen im Haar verteilt. Auch hier ist eine gezielte Behandlung einzelner Haarpartien nicht möglich. Außerdem werden bei Anwendung herkömmlicher Aerosol-Sprays und Aerosol-Schäume die behandelten Haare jeweils in ihrer ganzen Länge mit einer polymerhaltigen Zusammensetzung benetzt. Dies führt dazu, dass sich das Haar nicht mehr natürlich anfühlt. Das Anfühlen des Haares wird dann durch die Natur des jeweiligen, auf dem Haar befindlichen Polymerfilms bestimmt und das Haar wird durch das anhaftende Polymer belastet, was sich insbesondere bei feinem Haar negativ bemerkbar machen kann. Häufig beobachtete, unerwünschte Nebeneffekte sind, dass das behandelte Haar einen zu rauhen Griff, eine zu hohe Belastung oder eine ungenügende Elastizität aufweist oder sich zuviele sichtbare Rückstände auf dem Haar bilden.

Es bestand daher die Aufgabe, eine Möglichkeit zur Verfügung zu stellen, um gezielt einzelnen Haarpartien mehr Volumen und Standfestigkeit zu verleihen, ohne dabei die Haare zu belasten oder ihnen einen unnatürlich rauhen Griff zu verleihen.

Es wurde nun gefunden, dass die Aufgabe gelöst wird durch ein Haarbehandlungsprodukt zur Festigung des Haaransatzes, bestehend aus einer druckfesten Aerosolverpackung mit aufgesetztem Haarspray-Sprühkopf und einer in der Aerosolverpackung befindlichen Zusammensetzung, welche beim Versprühen einen schnell zusammenbrechenden, instabilen Schaum bildet und einen Gehalt aufweist an

(A) mindestens 60 Gew.% Wasser,
(B) mindestens einem schaumbrechenden Mittel,
(C) mindestens einem Tensid,
(D) mindestens einem stark haarfestigenden Polymer, ausgewählt aus Polymeren, welche eine grössere Wellstabilität aufweisen als Polyvinylpyrrolidon, gemessen nach 23 Stunden bei 20°C und 85% relativer Luftfeuchte und
(E) mindestens einem Aerosoltreibmittel.

Mit dem erfindungsgemäßen Produkt ist es möglich, insbesondere, wenn ein Sprühkopf mit engem Sprühwinkel gewählt wird, gezielt den Haaransatz zu besprühen. Hierbei wird am Haaransatz ein instabiler Schaum erzeugt, welcher als kurzzeitiger Indikator für die behandelte Haarpartie dienen kann. Der Schaum bricht auf dem Haaransatz schnell zusammen, ohne dass eine Einarbeitung, d.h. ein Kontakt mit den Händen erforderlich wäre, wie es bei herkömmlichen Haarschäumen der Fall ist. Das Zusammenbrechen des Schaumes wird vorteilhafterweise durch die Verwendung eines Haartrockners beschleunigt.

Haarfestigendes Polymer

Als Komponente (D) der Zusammensetzung werden stark haarfestigende Polymere eingesetzt. Polymere, welche eine besonders starke Festigung der Haare bewirken, weisen häufig den Nachteil auf, dass sie dem Haar einen unnatürlichen, unflexiblen und rauhen Griff verleihen, wenn sie in herkömmlichen Haarsprays oder Haarschäumen eingesetzt werden und dabei in der Regel auf das gesamte Haar aufgebracht werden. Derartige Polymere sind aber in den erfindungsgemäßen Produkten besonders gut geeignet. Da der Volumeneffekt durch eine Festigung ausschließlich am Haaransatz, d.h. an einem relativ kleinen Teil des Haares in der Nähe der Kopfhaut erfolgt, sind besondere Anforderungen an die Qualität der Festigung bzw. des Polymerfilms zu stellen, da sonst der lange Rest des Haares quasi wie ein Hebel wirkt und den gewünschten Effekt zunichte macht. Besonders gut geeignet sind daher Polymere, welche besonders feste, wenig flexible oder rauhe Filme bilden, d.h. gerade solche Polymere, die in herkömmlichen Haarsprays nachteilig sind.

Stark festigend im Sinne der Erfindung sind solche Polymere, die eine stärkere Festigung aufweisen als die Standard-Festigerpolymere Polyvinylpyrrolidon oder Vinylpyrrolidon/Vinylacetat Copolymer. Hierbei wird die Festigung bestimmt durch Wellstabilitätsmessungen, gemessen nach 23 Stunden bei 20°C und 85% relativer Luftfeuchte. Die Einzelheiten sind den Beispielen zu entnehmen. Bevorzugt sind Polymere, welche bei den genannten Bedingungen eine um mindestens 20%, besonders bevorzugt eine um mindestens 40% größere Wellstabilität gegenüber unbehandeltem Haar aufweisen.

Die festigenden Polymere (D) werden in einer Menge von vorzugsweise 0,1 bis 20, besonders bevorzugt von 1 bis 5 Gew.% eingesetzt.

Geeignete festigende Polymere sind z.B. Polymere, deren Herstellung in der WO 99/67216 beschrieben werden. Hierbei handelt es sich um Polymere, welche wiederkehrende Einheiten enthalten von alpha-Olefin-N-alkylmaleimid oder von alpha-Olefin-N-hydroxyalkyl-maleimid. Geeignet ist insbesondere ein imidiertes Poly(isobutylen-comaleinsäureanhydrid), ein Polymer mit der INCI-Bezeichnung Isobutylene/Ethylmaleimide/Hydroxyethylmaleimide Copolymer. Ein geeignetes Handelsprodukt ist Aquaflex® FX-64 der Firma ISP.

Geeignete festigende Polymere sind auch amphotere Copolymere, gebildet aus Alkylacrylamid, Alkylaminoalkylmethacrylat und zwei oder mehr Monomeren ausgewählt aus Acrylsäure, Methacrylsäure oder deren Estern, von denen mindestens eines der Monomere eine Säuregruppe enthält. Die Alkylgruppen können 1 bis 10 C-Atome enthalten. Geeignet ist insbesondere ein Copolymer gebildet aus Octylacrylamid, t-Butylaminoethylmethacrylat und zwei oder mehr Monomeren bestehend aus Acrylsäure, Methacrylsäure oder deren Ester, von denen mindestens eines der Monomere eine Säuregruppe enthält, ein Polymer mit der INCI-Bezeichnung Octylacrylamide/Acrylates/Butylaminoethylmethacrylate Copolymer. Ein entsprechendes Handelsprodukt ist Amphomer® oder Amphomer® LV-71 der Firma NATIONAL STARCH.

Weitere geeignete Polymere sind Copolymere aus Vinylpyrrolidon und quaternisiertem Dialkylaminoalkylacrylat oder quaternisiertem Dialkylaminoalkylmethacrylat. Die Alkylgruppen können 1 bis 4-C-Atome enthalten. Geeignet ist insbesondere ein Copolymer gebildet aus Vinylpyrrolidon und quaternisiertem Dimethylaminoethylmethacrylat, ein Polymer mit der INCI-Bezeichnung Polyquaternium-11. Entsprechende Handelsprodukte sind Gafquat® 755 N der Firma ISP oder Luviquat® PQ11 der Firma BASF.

Weitere geeignete Polymere sind Copolymere von Vinylacetat und Crotonsäure mit der INCI-Bezeichnung VA/Crotonates Copolymer. Ein entsprechendes Handelsprodukt ist Luviset® CA66 der Firma BASF.

Weitere geeignete Polymere sind Copolymere von Acrylamiden und einem oder mehreren Monomeren ausgewählt aus Acrylsäure, Methacrylsäure und deren einfachen Estern, insbesondere den C1- bis C4-Alkylestern, wobei vorzugsweise mindestens ein Monomer eine Carbonsäuregruppe enthält. Bevorzugte Acrylamide sind N-C1- bis C4-Alkylacrylamide. Die INCI-Bezeichnung ist Acrylates/Acrylamide Copolymer. Bevorzugt ist ein Acrylsäure/Ethylacrylat/N-tert.-Butylacrylamid Terpolymer. Ein entsprechendes Handelsprodukt ist Ultrahold® 8 der Firma BASF.

Weitere geeignete Polymere sind Terpolymere aus Vinylpyrrolidon, Vinylcaprolactam und einem basischen Acrylamidmonomer, vorzugsweise Dialkylaminoalkylmethacrylamid und Dialkylaminoalkylacrylamid, wobei die Alkylgruppen aus 1 bis 4 C-Atomen bestehen. Bevorzugt ist ein Vinylpyrrolidon/Vinylcaprolactam/Dimethylaminopropylmethacrylamid Terpolymer. Ein entsprechendes Handelsprodukt ist Aquaflex® SF40 der Firma ISP mit der INCI-Bezeichnung PVP/Vinylcaprolactam/DMAPA Acrylates Copolymer.

Weitere geeignete Polymere sind Copolymere aus zwei oder mehr verschiedenen Monomeren ausgewählt aus Acrylsäure, Methacrylsäure und deren einfachen Estern, insbesondere den C1- bis C4-Alkylestern, wobei vorzugsweise mindestens eines der Monomere eine Säuregruppe enthält. Die INCI-Bezeichnung ist Acrylates Copolymer. Bevorzugt ist ein t-Butylacrylat/Ethylacrylat/Methacrylsäure Terpolymer. Ein entsprechendes Handelsprodukt ist Luvimer® 100P der Firma BASF.

Tenside

Die Zusammensetzung des erfindungsgemäßen Produktes enthält als Komponente (C) mindestens ein Tensid, bei dem es sich vorzugsweise um ein schaumbildendes Tensid handelt. Schaumbildend sind solche Tenside, die im bei 20°C durchgeführten Ross-Miles Test eine Schaumhöhe von mindestens 1 cm ergeben. Die Tenside können nichtionischen, anionischen, kationischen oder amphoteren Charakter haben. Bevorzugt sind insbesondere nichtionische, schaumerzeugende Tenside. Die Tenside können einzeln oder in einem Gemisch eingesetzt werden. Die Menge an Tensiden kann variieren und ist so gewählt, dass sich eine für die Anwendungszwecke ausreichende Menge eines schnell zusammenbrechenden Schaumes bildet, wenn die Zusammensetzung aus der Aerosolverpackung ausgebracht wird. Die Tensidmenge beträgt typischerweise von 0,01 bis 5, bevorzugt von 0,1 bis 2 Gew.%.

Geeignete nichtionische Tenside sind beispielsweise C8-bis C18-Fettalkohole, die mit 3 bis 45 mol Ethylenoxid ethoxyliert sind, z.B. mit bis zu 40 Mol Ethylenoxid pro Mol Fettalkohol oxethylierter Lauryl-, Tetradecyl-, Cetyl-, Oleyl- oder Stearylalkohol, allein oder im Gemisch; hydriertes, mit 8 bis 45 mol Ethylenoxid ethoxyliertes Rizinusöl, C8- bis C18-Fettsäurealkanolamide; die Fettalkohole von oxethyliertem Lanolin oder oxethyliertes Lanolin; Polyglycerylether von gesättigten oder ungesättigten Fettalkoholen und Alkylphenolen mit 8 bis 30 C-Atomen im Alkylrest und 1 bis 10 Glyceryleinheiten im Molekül; Polyethylen/Polypropylen Blockcopolymere und oxethylierte Sorbitanfettsäureester. Besonders bevorzugt als nichtionische Tenside sind außerdem Alkylpolyglykoside, wobei die Alkylgruppen 8 bis 18 C-Atome enthalten können.

Geeignete anionische Tenside sind z.B. Alkylsulfate, Alkylethersulfate, Alkylsulfonate, Alkoylsarcosinate, Alkylisethionate oder Dialkylsulfosuccinate, wobei die Alkylgruppen 8 bis 18 C-Atome enthalten können.

Geeignete amphotere Tenside sind insbesondere solche vom Betaintyp. Beispiele für Betaine umfassen $C_8$- bis $C_{18}$-Alkylbetaine, wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Laurylbis-(2-hydroxypropyl)alphacarboxyethylbetain; $C_8$- bis $C_{18}$-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die $C_8$- bis $C_{18}$-Alkyldimethylammoniumacetate, die $C_8$- bis $C_{18}$-Alkyldimethylcarboxylmethylammoniumsalze sowie die $C_8$- bis $C_{18}$-Fettsäurealkylamidobetaine, wie z.B. das Kokosfettsäureamidopropylbetain und das N-Kokosfettsäureamidoethyl-N-[2-(carboxymethoxy)ethyl]glycerin (CTFA-Name: Cocoamphocarboxyglycinate).

Geeignete kationaktive Tenside sind Tenside, welche eine quaternäre Ammoniumgruppe enthalten. Geeignete kationische Tenside können durch die allgemeine Formel (I) dargestellt werden,

$$N^{(+)}R^1R^2R^3R^4 \; X^{(-)} \tag{I}$$

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens einer der Reste R1 bis R4 mindestens 8 C-Atome aufweist und X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise weitere Aminogruppen enthalten.

Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt ist Cetyltrimethylammoniumchlorid.

## Schaumbrechendes Mittel

Die Zusammensetzung des erfindungsgemäßen Produktes enthält mindestens ein schaumbrechendes Mittel (B), um ein genügend schnelles zusammenbrechen des unmittelbar nach dem Sprühen gebildeten Schaumes sicherzustellen. Geeignete schaumbrechende Mittel sind C1- bis C5-Alkohole, Öle und Silikonöle, z.B. lineare oder cyclische Dimethlysiloxane mit den INCI-Bezeichnungen Dimethicone oder Cyclomethicone. Geeignet sind auch nicht-organische Entschäumer, insbesondere solche auf Basis von physiologisch indifferenten Wirkkomponenten wie Polydimethylsiloxan/Silica. Derartige Wirkstoffe sind bekannt unter der Bezeichnung Simethicone. Hierbei handelt es sich um eine Mischung von Dimethiconen (Polydimethylsiloxanen) einer mittleren Kettenlänge von 200 bis 350 Dimethylsiloxaneinheiten und hydratisiertem Silica. Sie können eingesetzt werden in Form von Silicon-Antischaumemulsionen. Hierbei handelt es sich um wässrige Emulsionen von Simethiconen. Besonders bevorzugt sind C2- bis C4-Alkohole, insbesondere Ethanol und Isopropanol. Die Einsatzmengen betragen üblicherweise 1 bis maximal 30 Gew.%, vorzugsweise 5 bis 20 Gew.%. Stark wirksame Entschäumer wie die Simethicone können auch in geringeren Mengen eingesetzt werden, z.B. von 0,001 bis 1, vorzugsweise von 0,01 bis 0,5 Gew.%.

## Lösungsmittel

Das Lösungsmittel der Zusammensetzung des erfindungsgemäßen Produktes ist entweder Wasser oder ein Gemisch aus Wasser und einem oder mehreren organischen Co-Solventien. Der Gehalt an Wasser beträgt dabei mindestens 60 Gew.% bezogen auf die Gesamtzusammensetzung. Organische Co-Solventien können die oben genannten niederen Alkohole mit 1 bis 5, vorzugsweise 1 bis 4 C-Atomen wie z.B. Ethanol und Isopropanol sein. Typische weitere organische Lösungsmittel sind z.B. unverzweigte oder verzweigte Kohlenwasserstoffe wie Pentan, Hexan, Isopentan und cyclische Kohlenwasserstoffe wie Cyclopentan und Cyclohexan sowie hydrophile Lösungsmittel wie z. B. Glycerin, Ethylenglykol oder Propylenglykol. Die organischen Co-Solventien können in einer Menge bis maximal 30 Gew.%, vorzugsweise 0,1 bis 15, bevorzugt von 1 bis 10 Gew.% enthalten sein.

### Treibmittel

Das Aerosoltreibmittel (E) ist in der Zusammensetzung des erfindungsgemäßen Produktes vorzugsweise in einer Menge von 1 bis 30, besonders bevorzugt von 2 bis 20 Gew.% bezogen auf die Gesamtzusammensetzung enthalten. Als Treibmittel sind niedere Alkane, wie z.B. n-Butan, i-Butan, Propan, Butan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie z.B. $N_2$, $N_2O$ und $CO_2$ sowie Gemische der vorstehend genannten Treibmittel geeignet. Besonders bevorzugt sind die niederen Alkane und Dimethylether. Besonders gute Schaum- und Spheigenschaften werden erzielt mit einem Gemisch aus mindestens einem niederen Alkan, insbesondere Butan sowie Dimethylether. Bevorzugt ist dabei ein Verhältnis von Dimethylether zu Alkan von 2:1 bis 8:1, besonders bevorzugt von 3:1 bis 6:1.

### Zusätzliche Polymere

In einer weiteren Ausführungsform enthält die Zusammensetzung des erfindungsgemäßen Produktes zusätzlich mindestens ein weiteres, schwächer haarfestigendes Polymer und/oder mindestens ein verdickend wirkendes Polymer. Bei dem zusätzlichen, schwächer festigenden Polymer kann es sich z.B. um Polyvinylpyrrolidon oder um Polyvinylpyrrolidon/Vinylacetat Copolymere handeln. Verdickend wirkende Polymere sind z.B. Polyacrylate (INCI-Bezeichnung: Carbomer). Die zusätzlichen Polymer sind typischerweise in einer Menge von 0,01 bis 15, vorzugsweise von 0,5 bis 10 Gew.% enthalten.

### Optionale Zusatzbestandteile

Die Zusammensetzung des erfindungsgemäßen Produktes kann darüberhinaus die für Haarbehandlungsmittel üblichen Zusatzbestandteile enthalten, z.B. Parfüm- und Duftstoffe in einer Menge von 0,01 bis 0,5 Gew.%; Konservierungsstoffe, insbesondere bakterizide und fungizide Wirkstoffe in einer Menge von 0,01 bis 1,0 Gew.%; Puffersubstanzen, wie z.B. Natriumcitrat oder Natriumphosphat in einer Menge von 0,1 bis 1,0 Gew.%; Haarpflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; Glanzgeber, Vitamine, Kämmbarkeitsverbesserer und rückfettende Agenzien.

### Verpackung

Die Zusammensetzung des erfindungsgemäßen Produktes wird in einer druckfesten Aerosolverpackung abgefüllt. Als Verpackungsmaterial sind herkömmliche Materialien aus Metall oder druckfestem Kunststoff geeignet, insbesondere Weissblech und Aluminium. Dabei ist insbesondere bei Weissblech aufgrund des Wassergehaltes auf einen ausreichenden Korrosionsschutz zu achten, beispielsweise durch eine korrosionsbeständige Innenbeschichtung oder durch den Zusatz bekannter Korrosionsinhibitoren.

### Sprühkopf

Die befüllte Aerosolverpackung wird mit einem Haarspray-Sprühkopf versehen. Der Sprühkopf ist zur Sicherstellung einer gezielten Behandlung einzelner Haaransatzpartien idealerweise so ausgelegt, dass sich ein relativ enger Sprühwinkel von kleiner 40°, vorzugsweise maximal 30° ergibt. Hierfür können handelsübliche Sprühköpfe verwendet werden. Alternativ kann der Sprühkopf auch mit einer rohrförmigen Düse versehen werden.

### Anwendung

Bei einer typischen Anwendung des erfindungsgemäßen Produktes werden Kopfhaare strähnchenweise per Hand hochgenommen und im Ansatzbereich mit der erfindungsgemäßen Zusammensetzung besprüht. Dabei bildet sich auf dem besprühten Haaransatz ein instabiler Schaum. Zur Beschleunigung des Zusammenbrechens des Schaums und der Aushärtung des am Haaransatz abgeschiedenen Polymerfilms kann der besprühte Bereich mit einem Haartrockner getrocknet werden. Weitere Haarpartien werden in gleicher Weise behandelt. Zum Schluss werden die Haare gegebenenfalls durchgebürstet.

Die nachfolgenden Beispiele sollen den Gegenstand der Erfindung näher erläutern.

**Beispiele**

Die Auswahl an geeigneten stark haarfestigenden Polymeren erfolgt idealerweise anhand von Wellstabilitätsmessungen bei hoher Luftfeuchtigkeit im Vergleich zu Polyvinylpyrrolidon und Vinylpyrrolidon/Vinylacetat Copolymeren.

Hierzu werden pro zu untersuchender Polymerlösung an drei gebleichten und gewaschenen Zählhaarsträhnen (Eurohaar, 16,5 cm lang, 100 Haare pro Strähne) Wasserwellen erzeugt. Die Haarsträhnen werden mit 10 g Gewicht am unteren Ende beschwert, auf Spiralwickler aufgewickelt und mit jeweils 100 µl der zu untersuchenden Zusammensetzung behandelt und über Nacht bei 20°C und 85% relativer Luftfeuchtigkeit getrocknet und akklimatisiert. Anschließend werden die Strähnen vorsichtig von den Wicklern genommen und mit 50 mg beschwert an einem Gestell im Klimaraum bei 20°C und 85% relativer Luftfeuchtigkeit ausgehängt. Die Länge der Strähnen wird vor dem Aushängen und nach 23 Stunden gemessen.

Es wurden die folgenden 5 Polymerlösungen untersucht, wobei der Festoffgehalt an Polymer jeweils 5 Gew. % betrug:

| A: | 12,5 g | Aquaflex® FX-64 (Isobutylene/Ethylmaleimide/Hydroxyethylmaleimide Copolymer, 40%ig in 27% Wasser/33% Ethanol; ISP) |
| | ad 100 g | Ethanol |
| B | 5 g | Amphomer® (Octylacrylamide/Acrylates/ Butylaminoethyl Methacrylate Copolymer; National Starch) |
| | 3,4 g | Wasser |
| | 0,86 g | Aminomethylpropanol |
| | ad 100 g | Ethanol |
| C | 25 g | Gafquat® 755N (Polyquaternium-11, 20%ig in Wasser) |
| | Ad 100 g | Ethanol |
| D | 10 g | Luviskol® VA37E (PVP/VA Copolymer, 50%ig in Ethanol; BASF) |
| | 3,4 g | Wasser |
| | ad 100 g | Ethanol |
| E | 5 g | Luviskol K80 Pulver (PVP; BASF) |
| | 3,4 g | Wasser |
| | ad 100 g | Ethanol |

Bei einer Vergleichssträhne F wurde eine Wasserwelle ohne Behandlung mit einer Polymerlösung erzeugt.

Die Wellstabilität W wurde berechnet nach

$$W = (L - L_t) / (L - L_0) * 100\%.$$

Hierbei bezeichnet L die Länge der nicht gewellten Strähne, $L_t$ bezeichnet die Länge der gewellten Strähne zum Zeitpunkt t = 23 Stunden und $L_0$ bezeichnet die Länge der gewellten Strähne zum Zeitpunkt t=0.

Die Ergebnisse sind in der folgenden Tabelle zusammengefasst:

| Muster | Wellstabilität |
|--------|----------------|
| A | 78,7 ± 3,3 |
| B | 78,4 ± 7,5 |
| C | 63,7 ± 8,2 |
| D | 55,4 ± 3,4 |
| E | 58,6 ± 1,2 |
| F | 51,5 ± 2,3 |

| Beispiel 1: Aerosol-Sprühschaum zur Ansatzfestigung | |
|---|---|
| Aquaflex® FX-64 (Isobutylene/Ethylmaleimide/ Hydroxyethylmaleimide Copolymer, 40%ig in 27% Wasser/33% Ethanol; ISP) | 5,6 g |
| Cetyltrimethylammoniumchlorid | 0,15 g |
| Laureth-4 | 0,2 g |
| Parfüm | 0,2 g |
| Ethanol | 10 g |
| Wasser | Ad 100 g |

Die Wirkstoffmischung wurde im Verhältnis 80:20 mit Dimethylether/Butan (4:1) als Treibmittel in einer Aerosoldose aus Aluminium abgefüllt und mit einem Sprühkopf (DPV APSL 0,025", 0,64 mm) versehen.

| Beispiel 2: Aerosol-Sprühschaum zur Ansatzfestigung | |
|---|---|
| Aquaflex® FX-64 (Isobutylene/Ethylmaleimide/ Hydroxyethylmaleimide Copolymer, 40%ig in 27% Wasser/33% Ethanol; ISP) | 10 g |
| Oramix® NS10 (Decylglucosid, 55%ig in Wasser) | 0,1 g |
| Laureth-4 | 0,2 g |
| Parfüm | 0,2 g |
| Ethanol | 10 g |
| Wasser | Ad 100 g |

Die Wirkstoffmischung wurde im Verhältnis 80:20 mit Dimethylether/Butan (4:1) als Treibmittel in einer Aerosoldose aus Aluminium abgefüllt und mit einem Sprühkopf (DPV APSL 0,020", 0,51 mm) versehen.

| Beispiel 3: Aerosol-Sprühschaum zur Ansatzfestigung | |
|---|---|
| Aquaflex® FX-64 (Isobutylene/Ethylmaleimide/ Hydroxyethylmaleimide Copolymer, 40%ig in 27% Wasser/33% Ethanol; ISP) | 5,6 g |
| Vinylacetat/Crotonsäure Copolymer | 0,6 g |
| Aminomethylpropanol (95%ig) | 0,1 g |
| Cetyltrimethylammoniumchlorid | 0,1 g |
| Laureth-4 | 0,1 g |
| Parfüm | 0,2 g |
| Ethanol | 20 g |
| Wasser | Ad 100 g |

Die Wirkstoffmischung wurde im Verhältnis 80:20 mit Dimethylether/Butan (4:1) als Treibmittel in einer Aerosoldose aus Aluminium abgefüllt und mit einem Sprühkopf (DPV APSL 0,020", 0,51 mm) versehen.

| Beispiel 4: Aerosol-Sprühschaum zur Ansatzfestigung | |
|---|---|
| Aquaflex® FX-64 (Isobutylene/Ethylmaleimide/ Hydroxyethylmaleimide Copolymer, 40%ig in 27% Wasser/33% Ethanol; ISP) | 7 g |
| Cetyltrimethylammoniumchlorid | 0,15 g |
| Laureth-4 | 0,2 g |
| Parfüm | 0,2 g |

(fortgesetzt)

| Beispiel 4: Aerosol-Sprühschaum zur Ansatzfestigung | |
|---|---|
| Ethanol | 30 g |
| Wasser | Ad 100 g |

Die Wirkstoffmischung wurde im Verhältnis 80:20 mit Dimethylether/Butan (4:1) als Treibmittel in einer Aerosoldose aus Aluminium abgefüllt und mit einem Sprühkopf (DPV APSL 0,020", 0,51 mm) versehen.

**Patentansprüche**

1. Haarbehandlungsprodukt zur Festigung des Haaransatzes, bestehend aus einer druckfesten Aerosolverpackung mit aufgesetztem Haarspray-Sprühkopf und einer in der Aerosolverpackung befindlichen Zusammensetzung, welche beim Versprühen einen schnell zusammenbrechenden, instabilen Schaum bildet und einen Gehalt aufweist an

   (A) mindestens 60 Gew.% Wasser,
   (B) mindestens einem schaumbrechenden Mittel,
   (C) mindestens einem Tensid,
   (D) mindestens einem stark haarfestigenden Polymer, ausgewählt aus Polymeren, welche eine grössere Wellstabilität aufweisen als Polyvinylpyrrolidon, gemessen nach 23 Stunden bei 20°C und 85% relativer Luftfeuchte und
   (E) mindestens einem Aerosoltreibmittel.

2. Produkt nach Anspruch 1, **dadurch gekennzeichnet, dass** das schaumbrechende Mittel (B) ausgewählt ist aus C1-bis C5-Monoalkoholen und in einer Menge von maximal 30 Gew.% eingesetzt wird.

3. Produkt nach einem der vorhergehenden Ansprüche, **dadurch gekennzeichnet, dass** das Polmyer (D) ausgewählt ist aus

   - Polymeren, welche wiederkehrende Einheiten enthalten von alpha-Olefin-N-alkylmaleimid und/oder von alpha-Olefin-N-hydroxyalkyl-maleimid,
   - Copolymeren, gebildet aus Alkylacrylamid, Alkylaminoalkylmethacrylat und zwei oder mehr Monomeren ausgewählt aus Acrylsäure, Methacrylsäure oder deren Estern,
   - Copolymeren aus Vinylpyrrolidon und quaternisiertem Dialkylaminoalkylmethacrylat,
   - Copolymeren von Vinylacetat und Crotonsäure,
   - Copolymeren von Acrylamiden und einem oder mehreren Monomeren ausgewählt aus Acrylsäure, Methacrylsäure und deren einfachen Estern,
   - Terpolymeren aus Vinylpyrrolidon, Vinylcaprolactam und einem basischen Acrylamidmonomer,
   - Copolymeren aus zwei oder mehr verschiedenen Monomeren ausgewählt aus Acrylsäure, Methacrylsäure und deren einfachen Estern.

4. Produkt nach Anspruch 3, **dadurch gekennzeichnet, dass** das Polymer (D) ein Copolymer aus Isobutylen-N-Ethylmaleimid und Isobutylen-N-Hydroxyethyl-maleimid ist.

5. Produkt nach einem der vorhergehenden Ansprüche, **dadurch gekennzeichnet, dass** das Aerosoltreibmittel (E) ausgewählt ist aus Mischungen von Dimethylether mit mindestens einem Kohlenwasserstoff ausgewählt aus Propan, n-Butan, Isobutan und Pentan.

6. Produkt nach Anspruch 5, **dadurch gekennzeichnet, dass** das Gewichtsverhältnis von Dimethylether zu Kohlenwasserstoff 2:1 bis 8:1 beträgt.

7. Produkt nach einem der vorhergehenden Ansprüche, **dadurch gekennzeichnet, dass** der Sprühkopf so ausgelegt ist, dass der Sprühwinkel weniger als 40° beträgt.

8. Produkt nach einem der vorhergehenden Ansprüche, **dadurch gekennzeichnet, dass** die Zusammensetzung zusätzlich mindestens ein weiteres haarfestigendes oder verdickendes Polymer enthält.

9. Produkt nach Anspruch 8, **dadurch gekennzeichnet, dass** das weitere haarfestigende oder verdickende Polymer ausgewählt ist aus Polyvinylpyrrolidon, Polyvinylpyrrolidon/Vinylacetat Copolymeren und Polyacrylaten.

10. Verwendung eines Produktes gemäß einem der Ansprüche 1 bis 9 zur Vergrößerung des Volumens einer Frisur oder zur gezielten Festigung des Haaransatzes.

11. Verfahren zur Vergrößerung des Volumens einer Frisur oder zur gezielten Festigung des Haaransatzes, wobei

(1.) Kopfhaare strähnchenweise per Hand hochgenommen werden,
(2.) anschließend die hochgenommenen Strähnchen im Ansatzbereich mit einem Produkt gemäß einem der Ansprüche 1 bis 9 besprüht werden,
(3.) anschließend die Strähnchen weiter hochgehalten und mit einem Haartrockner im besprühten Ansatzbereich getrocknet werden und
(4.) gegebenenfalls weitere Haarpartien in gleicher Weise behandelt werden und zum Schluss die Haare gegebenenfalls durchgebürstet werden.

**Claims**

1. Hair-treatment product for setting the roots of the hair, consisting of a pressure-resistant aerosol packaging with attached hairspray spray-head and a composition inside the aerosol packaging which, upon spraying, forms a very rapidly collapsing, unstable foam and has a content of

(A) at least 60% by weight of water,
(B) at least one foam-breaking agent,
(C) at least one surfactant,
(D) at least one strongly hair-setting polymer chosen from polymers which have greater wave stability than polyvinylpyrrolidone, measured after 23 hours at 20°C and 85% relative atmospheric humidity and
(E) at least one aerosol propellant.

2. Product according to Claim 1, **characterized in that** the foam-breaking agent (B) is chosen from C1- to C5-monoalcohols and is used in an amount of at most 30% by weight.

3. Product according to one of the preceding claims, **characterized in that** the polymer (D) is chosen from

- polymers which contain repeat units of alpha-olefin-N-alkylmaleimide and/or of alpha-olefin-N-hydroxyalkylmaleimide,
- copolymers formed from alkylacrylamide, alkylaminoalkyl methacrylate and two or more monomers chosen from acrylic acid, methacrylic acid or esters thereof,
- copolymers of vinylpyrrolidone and quaternized dialkylaminoalkyl methacrylate,
- copolymers of vinyl acetate and crotonic acid,
- copolymers of acrylamides and one or more monomers chosen from acrylic acid, methacrylic acid and monoesters thereof,
- terpolymers of vinylpyrrolidone, vinyl-caprolactam and a basic acrylamide monomer,
- copolymers of two or more different monomers chosen from acrylic acid, methacrylic acid and monoesters thereof.

4. Product according to Claim 3, **characterized in that** the polymer (D) is a copolymer of isobutylene-N-ethylmaleimide and isobutylene-N-hydroxyethylmaleimide.

5. Product according to one of the preceding claims, **characterized in that** the aerosol propellant (E) is chosen from mixtures of dimethyl ether with at least one hydrocarbon chosen from propane, n-butane, isobutane and pentane.

6. Product according to Claim 5, **characterized in that** the weight ratio of dimethyl ether to hydrocarbon is 2:1 to 8:1.

7. Product according to one of the preceding claims, **characterized in that** the spray-head is designed such that the spray angle is less than 40°.

8. Product according to one of the preceding claims, **characterized in that** the composition additionally comprises at least one further hair-setting or thickening polymer.

9. Product according to Claim 8, **characterized in that** the further hair-setting or thickening polymer is chosen from polyvinylpyrrolidone, polyvinylpyrrolidone/vinyl acetate copolymers and polyacrylates.

10. Use of a product according to one of Claims 1 to 9 for increasing the volume of a hairstyle or for the targeted setting of the roots of the hair.

11. Method of increasing the volume of a hairstyle or for the targeted setting of the roots of the hair, where

(1.) head hair is lifted in tresses by hand,
(2.) then the lifted tresses are sprayed in the roots area with a product according to one of Claims 1 to 9,
(3.) then the tresses are held up further and are dried in the sprayed roots area using a hairdryer and
(4.) where necessary, further sections of hair are treated in the same way and, finally, the hair is brushed through, if desired.

**Revendications**

1. Produit de traitement capillaire destiné à fixer la racine du cheveu, constitué d'un emballage aérosol tenant la pression, surmonté d'une tête de pulvérisation pour laque pour cheveux, et d'une composition se trouvant dans l'emballage aérosol, qui forme lors de la pulvérisation une mousse instable s'affaissant rapidement et contient

(A) au moins 60 % en poids d'eau,
(B) au moins un agent provoquant l'affaissement de mousse,
(C) au moins un tensioactif,
(D) au moins un polymère fixant fortement le cheveu, choisi parmi des polymères qui présentent une plus grande tenue de l'ondulation que la polyvinylpyrrolidone, mesurée après 23 heures à 20°C et 85 % d'humidité relative de l'air et
(E) au moins un propulseur pour aérosol.

2. Produit selon la revendication 1, **caractérisé en ce que** l'agent provoquant l'affaissement de mousse (B) est choisi parmi des monoalcools en $C_1$-$C_5$ et est utilisé en une quantité d'au maximum 30 % en poids.

3. Produit selon l'une quelconque des revendications précédentes, **caractérisé en ce que** le polymère (D) est choisi parmi

- des polymères qui contiennent des motifs répétitifs d'$\alpha$-oléfine-N-alkylmaléimide et/ou d'$\alpha$-oléfine-N-hydroxyalkyl-maléimide,
- des copolymères formés à partir d'alkylacrylamide, de méthacrylate d'alkylaminoalkyle et de deux ou plus de deux monomères choisis parmi l'acide acrylique, l'acide méthacrylique ou leurs esters,
- des copolymères de vinylpyrrolidone et méthacrylate de dialkylaminoalkyle rendu quaternaire,
- des copolymères d'acétate de vinyle et acide crotonique,
- des copolymères d'acrylamides et d'un ou plusieurs monomères choisis parmi l'acide acrylique, l'acide méthacrylique et leurs monoesters,
- des terpolymères de vinylpyrrolidone, vinylcaprolactame et d'un monomère acrylamide basique,
- des copolymères de deux différents monomères ou plus, choisis parmi l'acide acrylique, l'acide méthacrylique et leurs monoesters.

4. Produit selon la revendication 3, **caractérisé en ce que** le polymère (D) est un copolymère d'isobutylène-N-éthylmaléimide et isobutylène-N-hydroxyéthyl-maléimide.

5. Produit selon l'une quelconque des revendications précédentes, **caractérisé en ce que** le propulseur pour aérosol (E) est choisi parmi des mélanges d'éther diméthylique et d'au moins un hydrocarbure choisi parmi le propane, le n-butane, l'isobutane et le pentane.

**6.** Produit selon la revendication 5, **caractérisé en ce que** le rapport pondéral de l'éther diméthylique à l'hydrocarbure va de 2:1 à 8:1.

**7.** Produit selon l'une quelconque des revendications précédentes, **caractérisé en ce que** la tête de pulvérisation est configurée de telle façon que l'angle de pulvérisation est inférieur à 40°.

**8.** Produit selon l'une quelconque des revendications précédentes, **caractérisé en ce que** la composition contient en outre au moins un autre polymère fixant le cheveu ou épaississant.

**9.** Produit selon la revendication 8, **caractérisé en ce que** l'autre polymère fixant le cheveu ou épaississant est choisi parmi la polyvinylpyrrolidone, des copolymères polyvinylpyrrolidone/acétate de vinyle et les polyacrylates.

**10.** Utilisation d'un produit selon l'une quelconque des revendications 1 à 9, pour augmenter le volume d'une coiffure ou pour fixer dans un but précis la racine du cheveu.

**11.** Procédé pour augmenter le volume d'une coiffure ou pour fixer dans un but précis la racine du cheveu, dans lequel

(1.) on relève à la main les cheveux par mèches,
(2.) on pulvérise ensuite sur les mèches élevées, dans la zone de la racine, un produit selon l'une quelconque des revendications 1 à 9,
(3.) ensuite on continue à maintenir élevées le mèches et on les sèche avec un sèche-cheveux dans la zone de la racine ayant reçu la pulvérisation,
(4.) éventuellement on traite de la même façon d'autres parties de la chevelure et enfin les cheveux sont éventuellement brossés.